# EUROPÄISCHE PATENTSCHRIFT

(11) **EP 0 985 374 B1**
(45) Veröffentlichungstag und Bekanntmachung des Hinweises auf die Patenterteilung: **01.12.2004**
(21) Anmeldenummer: 99250309.4
(22) Anmeldetag: 03.09.1999
(51) Int. Cl.: A61B 5/11, A61N 1/365

(54) **Vorrichtung zur Erkennung der Körperlage eines Menschen**
Device for detecting a human body posture
Appareil de détection de la posture d'une personne

(30) Priorität: 08.09.1998 DE 19842107
(43) Veröffentlichungstag der Anmeldung: 15.03.2000
(73) Patentinhaber: BIOTRONIK Mess- und Therapiegeräte GmbH & Co Ingenieurbüro Berlin, 12359 Berlin (DE)
(72) Erfinder: Meyer, Wolfgang, 91056 Erlangen (DE)
(74) Vertreter: Eisenführ, Speiser & Partner

(56) Entgegenhaltungen:
- EP-A- 0 845 240
- EP-A- 0 911 063
- US-A- 4 926 863
- US-A- 5 354 317
- US-A- 5 466 245
- US-A- 5 676 690
- US-A- 5 797 840

## Beschreibung

Die Erfindung betrifft eine Vorrichtung zur Erkennung der Körperlage eines Menschen gemäß dem Oberbegriff des Anspruchs 1 und ferner ein eine solche Vorrichtung enthaltendes implantierbares Herztherapiegerät.

Es sind ratenadaptive Herzschrittmacher bekannt, die die adaptive Stimulationsrate anhand eines Vergleichs zwischen der aktuellen Sensor-Signalmorphologie und der bei ruhendem Patienten bestimmten Morphologie steuern. So wird beispielsweise beim Schrittmacher "Inos" der Anmelderin die jeweils aktuelle intrakardiale Impedanzkurve mit einer Referenzkurve des ruhenden Patienten verglichen und aus der Integraldifferenz die adaptive Rate ermittelt. Bei solchen Schrittmachern ist es wichtig, die Referenzkurve einerseits ständig aktuell zu halten, sie andererseits aber tatsächlich nur am ruhenden Patienten zu aktualisieren. Für diese automatische Aktualisierung der Referenzkurve ist eine Erkennung der Körperlage des Patienten (stehend bzw. sitzend oder liegend) wünschenswert, da die Lageerkennung in gewissen Grenzen auch die Erkennung von Ruhephasen des Patienten ermöglicht.

Bei ratenadaptiven Schrittmachern kann ein weiteres Problem darin liegen, daß die Signalmorphologie des zur Ratenadaption herangezogenen Meßsignals bei Lagewechseln des Patienten ebenfalls starke Veränderungen zeigt, ohne daß diese Ursache durch den Ratenadaptions-Algorithmus erkannt werden kann. Dies kann zu paradoxem Schrittmacherverhalten, wie beispielsweise einer kontraproduktiven Absenkung der Stimulationsrate beim Aufstehen des Patienten, führen. Durch eine unabhängige Lageerkennung könnte der Ratenadaptionsalgorithmus ein weiteres Eingangssignal erhalten, um auf Lagewechsel richtig reagieren zu können.

Nicht zuletzt könnte der korrekte Zeitpunkt für eine automatische Nachtabsenkung der Stimulationsrate ermittelt werden, indem die Zeitbasis des Schrittmachers unbeeinflußt von Zeitzonenüberschreitungen oder wechselndem Tagesrhythmus des Patienten anhand von dessen tatsächlichen Liege- bzw. Ruhephasen eingestellt wird; vgl. WO91/08017.

Es sind seit längerem Vorschläge bekannt, die Körperlage eines Patienten mit Hilfe von Quecksilber- oder ähnlichen lageempfindlichen Schaltern zu erfassen. Diese haben sich aber wegen verschiedener Probleme in der Praxis bislang nicht bewährt.

In der Grundlagenforschung wird als Indikator für das (durch einen Lagewechsel beeinflußte) sympathovagale Gleichgewicht die Herzratenvariabilität in den definierten Frequenzintervallen LF (0,05-0,15 Hz) und HF (0,15-0,4 Hz) herangezogen, vgl. S. Akselrod et al: "Hemodynamic regulation: investigation by spectral analysis", Am. J. Physiol. 249, H867-875 (1985). Dieses Verfahren ist im praktisch besonders wichtigen Einsatzgebiet der Schrittmacherpatienten nicht anwendbar, da die Variabilität der Herzrate bei Schrittmacherpatienten normalerweise nicht zur Verfügung steht.

Aus der US 5,797,840 ist eine Vorrichtung zur Analyse eines zeitabhängigen Leistungsspektrums eines physiologischen Signals, frequenzmoduliert durch das sympathovagale Nervensystem.

Der Erfindung liegt deshalb die Aufgabe zugrunde, ein auch für Schrittmacherpatienten geeignetes, zuverlässiges Vorrichtung zur Lageerkennung eines Menschen und eine Vorrichtung zur Durchführung dieses Verfahrens anzugeben.

Die Aufgabe wird durch eine Vorrichtung mit den Merkmalen des Anspruchs 1 gelöst.

Die Erfindung schließt die grundsätzliche Lehre ein, die Morphologie intrakardialer physiologischer Sensorsignale zur Lageerkennung (Orthostase, Wechsel zwischen stehend und liegend auszunutzen. Geeignet sind Sensorsignale, die eine Abhängigkeit vom sympathischen/parasympathischen Nervensystem aufweisen. Es können insbesondere hämodynamische Signale wie Impedanz oder Druck, aber auch elektrische Signale wie die VER (ventrikulär evozierte Reizantwort) verwendet werden.

Weiter schließt die Erfindung die Lehre ein, aus der Signalmorphologie eines einzelnen Herzzyklus einen bestimmten Signalparameter zu extrahieren. Dieser Signalparameter wird für eine Vielzahl von Herzzyklen bestimmt, und seine Schlag-zu-Schlag-Schwankungen werden ermittelt.

Die Schlag-zu-Schlag-Schwankungen werden einer Frequenzanalyse unterzogen, um die spektrale Leistungsdichte zu erhalten. Die spektrale Leistungsdichte stellt ein Maß für die Schlag-zu-Schlag-Variabilität des Signalparameters mit bestimmten Variationsfrequenzen dar. Eine hohe Leistungsdichte bei einer bestimmten Frequenz bedeutet eine hohe Variabilität mit eben dieser Variationsfrequenz. Bei stehendem Patienten ist das Verhältnis zwischen hohen (zwischen ca. 0,15 und 0,4 Hz liegenden) und niedrigen (zwischen etwa 0,05 und 0,15 Hz liegenden) Variationsfrequenzen von Signalen unter sympathischem/parasympathischem Einfluß niedrig, bei liegendem Patienten ist dieses Verhältnis dagegen hoch. Daher wird die spektrale Leistungsdichte jeweils über den hohen und einen niedrigen Frequenzbereich integriert, um dann aus dem Verhältnis der beiden Integrale die Lage des Patienten von Schlag zu Schlag ermitteln zu können.

In einer bevorzugten Ausführung wird die Schlag-zu-Schlag-Variabilität des intrakardialen Impedanzsignals zur Detektion von Positionswechseln des Patienten verwendet. Zur Messung des Impedanzsignals wird bevorzugt über eine unipolare Schrittmacherelektrode ein hochfrequenter Wechselstromimpuls mit einer mittleren Stromstärke von 1.3 mA eingespeist und der Spannungsabfall zwischen Elektrodenspitze und Schrittmachergehäuse gemessen.

Als geeigneter Parameter zur Konstruktion einer Zeitreihe aus dem Signalverlauf wird die Steigung des Impedanzsignals an einem festen Punkt während des Herzzyklus bestimmt. Analog zur oben erwähnten Analyse der Herzratenvariabilität werden insbesondere zeitabhängige Frequenzanalysen von Zeitreihen des aus dem Impedanzverlauf extrahierten Steigungsparameters durchgeführt. Um das gesamte Frequenzspektrum zeitaufgelöst zu untersuchen, wird die autoregressive Spektralanalyse (ARSA) verwendet. Hierbei wird ein lineares Modell an die gemessene Zeitreihe eines Parameters angepaßt und aus den Koeffizienten die spektrale Leistungsdichte berechnet. Dies geschieht bevorzugt in jedem Punkt der Zeitreihe mit einem autoregressiven Modell der Ordnung 20. Die berechnete spektrale Leistungsdichte wird jeweils in den Teilbereichen niederer Frequenzen (LF - Blutdruckvariabilität) und höherer Frequenzen (HF - Atemvariabilität) integriert und verglichen.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein Funktions-Blockschaltbild eines ratenadaptiven Herzschrittmachers als bevorzugtes Ausführungsbeispiel der Erfindung und
Figur 2 eine grafische Darstellung zur Verdeutlichung der Signifikanz der mit dem erfindungsgemäßen Verfahren erhaltenen Ergebnisse.

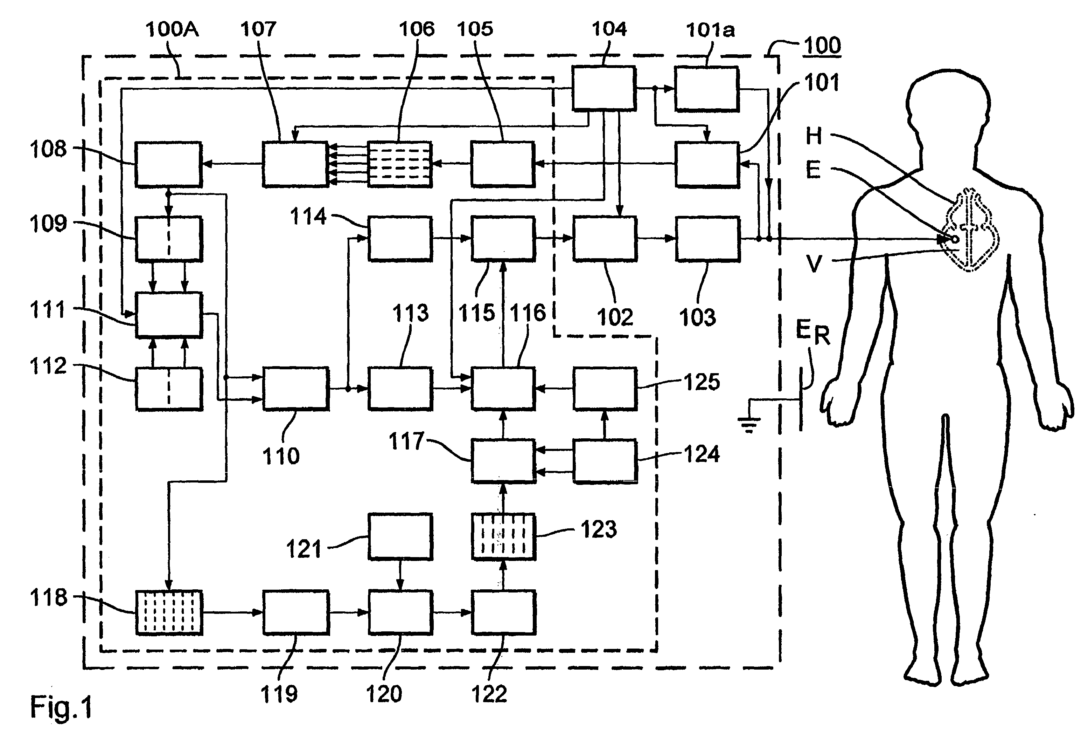

In Fig. 1 sind in Form eines Funktions-Blockschaltbildes wesentliche Komponenten eines ratenadaptiven Herzschrittmachers 100 gezeigt, wobei im Interesse der Übersichtlichkeit an sich bekannte, für die Ausführung der Erfindung jedoch nicht wesentliche Schrittmacherelemente fortgelassen sind.

Der Herzschrittmacher 100 ist eingangsseitig und auch ausgangsseitig mit einer intrakardialen Elektrode E im Ventrikel V des Herzens H eines Patienten verbunden. Mit der Elektrode E ist neben einer (als solche bekannten und hier nicht dargestellten) Herzsignal-Eingangsstufe zur Erfassung ventrikulärer Herzsignale eine Impedanzstufe verbunden. Diese umfaßt einen Abtaststromgenerator 101 a und eine Impedanzsignal-Eingangsstufe 101, die Filter- und Verstärkerstufen zur signalformgetreuen Erfassung eines intrakardialen Impedanzsignals aufweist. Durch einen Stimulationsimpulsgenerator 102 erzeugte Stimulationsimpulse werden über eine Ausgangsstufe 103 und die Elektrode E an den Ventrikel V abgegeben. Eine Gehäuseelektrode ER dient als Referenzelektrode für eine unipolare Signalerfassung und Stimulation.

Der Schrittmacher 100 umfaßt eine Ablaufsteuerung 104, die den gesamten Betriebsablauf steuert - in der Figur sind aber im Interesse der Übersichtlichkeit nur die Steuersignalverbindungen zu einigen im gegebenen Zusammenhang wichtigen Komponenten gezeigt. Im Zusammenhang mit der Erfindung ist wichtig, daß die Ablaufsteuerung 104 in zeitlicher Abstimmung auf die Ausgabe der Stimulationsimpulse den Abtaststromgenerator 101a sowie die Eingangs-Verarbeitung eines zeitabhängigen Impedanzsignals in einem vorbestimmten Zeitfenster nach dem Stimulus durch die Impedanzsignal-Eingangsstufe 101 steuert.

Die Erzeugung und Bereitstellung der Ratensteuersignale zur Steuerung der Stimulationsrate entsprechend dem physiologischen Bedarf des Patienten P erfolgt in einer eingangsseitig mit der Impedanzsignal-Eingangsstufe 101 und ausgangsseitig mit dem Stimulationsimpulsgenerator 102 verbundenen Ratenberechnungseinrichtung 100 A. Diese umfaßt eine über einen A/D-Wandler 105 mit der Impedanzsignal-Eingangsstufe 101 verbundene Impedanzsignal-Speichereinheit 106 mit einer Mehrzahl separater Speicherbereiche, in denen jeweils nach einem Stimulus ein vollständiges Impedanzsignal gespeichert wird. Mit der Speichereinheit 106 ist eine - ebenfalls durch die Ablaufsteuerung 104 gesteuerte - Kalibrationseinheit 107 verbunden.

In der Kalibrationseinheit 107 werden für einen vorbestimmten Zeitraum oder eine vorbestimmte Anzahl von Impedanzsignalen (entsprechend einem in der Ablaufsteuerung 104 gespeicherten Programm) einmalig oder in vorbestimmten größeren Abständen diejenigen "Zeitpunkte" (Zeitabstände zum Stimulus-Zeitpunkt) des Impedanzsignals ermittelt, für die die Differenz der Signalamplituden maximale Variabilität zeigt. In einer der Kalibrationseinheit 107 nachgeschalteten Arithmetikstufe 108 wird fortlaufend für das jeweils letzte aufgenommene Impedanzsignal Z(t) der aktuelle Quotient aus dem Amplitudendifferenzwert ΔZ = Z2(t2) - Z1(t1) zu diesen Zeitpunkten und der Differenz (t2 - t1) der Zeitpunkte gebildet, d.h. die Steigung der Impedanzkurve im Abschnitt höchster Variabilität ermittelt.

Ausgangsseitig ist die Arithmetikstufe 108 mit einem Signalparameterspeicher 109 sowie einer Adaptionsstufe 110 verbunden. Im Speicher 109 sind - nach dem FIFO-Prinzip fortlaufend aktualisiert - der jeweils erfaßte größte und kleinste Steigungswert festgehalten, und diese werden einem Eingangspaar einer Normierungsstufe 111 zugeführt, die über ein zweites Eingangspaar mit einem Ratengrenzwertspeicher 112 verbunden ist, in dem die für die Ratensteuerung minimal und maximal zulässige Stimulationsratenwerte gespeichert sind.

In der Normierungsstufe 111 wird aus dem jeweils vorliegenden Amplitudendifferenz-Minimal- und -Maximalwert und dem vorgegebenen Stimulationsraten-Minimal- und -Maximalwert der aktuell gültige "physiologische" speicher 124 verbunden ist und in der eine Bewertung des aktuellen Quotienten anhand von Vergleichswerten erfolgt. Die Vergleicher- und Bewertungseinheit 117 gibt schließlich ein die Lage des Patienten (stehend/sitzend/liegend) kennzeichendes Lagesignal an die Korrekturstufe 113 aus, das dort zur Ermittlung eines sekundären Ratensteuersignals gemäß einer in einem Korrekturprogrammspeicher 125 gespeicherten Korrekturvorschrift berechnet wird.

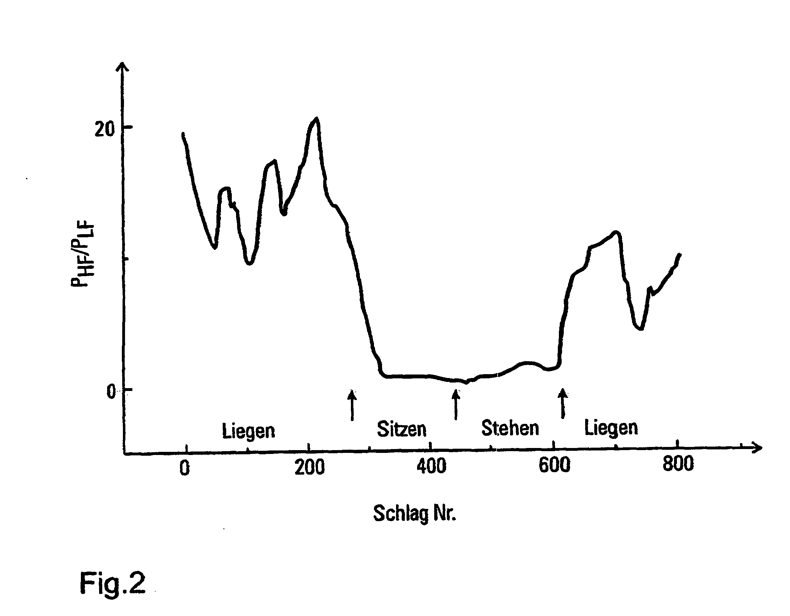

Fig. 2 verdeutlicht in Form einer über mehrere hundert Herzaktionen experimentell ermittelten Zeitkurve die Abhängigkeit des in der Arithmetikstufe 122 berechneten Quotienten der spektralen Leistungsdichten PHF/PLF von der Lage des Patienten und damit die Relevanz dieser Größe für die Gewinnung eines Korrektursignals für die Ratenadaption eines Schrittmachers.

Die Erfindung beschränkt sich in ihrer Ausführung nicht auf das vorstehend angegebene bevorzugte Ausführungsbeispiel. Vielmehr ist eine Anzahl von Varianten möglich, welche von der dargestellten Lösung auch in anders gearteten Ausführungen Gebrauch machen.

Die Erfassung der physiologischen Größe, aus der der relevante Signalparameter bestimmt wird, erfolgt nicht notwendigerweise auf elektrischem Wege über eine oder mehrere Elektrode(n), sondern kann auch auf mechanischem Wege, etwa mittels eines im Herzen oder einem herznahen Gefäß angeordneten Drucksensors, geschehen.

Die Bestimmung der spektralen Energiedichte kann neben dem ARSA-Verfahren auch mit eienm anderen an sich bekannten Korrelations- bzw. Transformationsverfahren erfolgen.

Die Auswertung der spektralen Energiedichte erfordert nicht notwendigerweise eine Verhältniswertbildung aus zwei Bereichen, sondern kann ggfs. mehrere Bereiche berücksichtigen oder aber sich nur auf einen Frequenzbereich erstrecken.

## Patentansprüche

1. Vorrichtung (100A) zur Erkennung der Körperlage eines Menschen, umfassend
einen Sensor (E, 101) zur morphologiegetreuen Erfassung eines physiologischen Signals, welcher im Herzen (H) oder einem herznahen Gefäß angeordnet werden kann,
**gekennzeichnet durch**
eine Steuereinheit (104) zur Ansteuerung des Sensors zu vorbestimmten Zeitpunkten, die mit einem Detektor (E) für ein physiologisches Ereignis oder einem Stimulationssignalerzeuger (102) verbunden und ausgebildet ist, ein von dem Sensor erfasstes Signal bezogen auf das physiologische Ereignis oder das Stimulationsereignis abzugreifen,
eine eingangsseitig mit dem Sensor verbundene erste Verarbeitungseinheit (105 bis 108) zur Bestimmung eines Wertes eines Signalparameters aus der Morphologie des erfassten Signals gemäß einer vorgegebenen Signalverarbeitungsvorschrift,
einen Signalparameterspeicher (118) zur Speicherung von in der Vergangenheit bestimmten Signalparameterwerten,
eine eingangsseitig mit dem Signalparameterspeicher verbundene zweite Verarbeitungseinheit (119) zur Ermittlung einer Schwankungskurve der gespeicherten Signalparameterwerte,
einen eingangsseitig mit der zweiten Verarbeitungseinheit verbundenen Frequenzanalysator (120) zur Gewinnung einer spektralen Leistungsdichte aus der Schwankungskurve,
eine eingangsseitig mit dem Frequenzanalysator verbundene dritte Verarbeitungseinheit (122, 123) zur Bestimmung des Verhältnisses der Werte der spektralen Leistungsdichte in mindestens zwei vorbestimmten Frequenzbereichen und
eine eingangsseitig mit der dritten Verarbeitungseinheit verbundene Vergleicher- und Bewertungseinheit (117) zur Ausgabe eines Lagesignals in Abhängigkeit vom ermittelten Verhältniswert.

2. Vorrichtung nach Anspruch 1, **dadurch gekennzeichnet, dass** der Sensor eine, (E), welche intrakardial angeordnet werden kann und eine zur Erfassung der Morphologie eines intrakardialen EKG- oder Impedanzsignals ausgebildete Filter- und Verstärkerstufe (101) aufweist.

3. Vorrichtung nach Anspruch 2, **dadurch gekennzeichnet, dass** die Elektrode eine unipolare Elektrode ist.

4. Vorrichtung nach einem der vorhergehenden Ansprüche, **dadurch gekennzeichnet, dass** das physiologische Signal die intrakardiale Impedanz oder die ventrikulär evozierte Reizantwort VER ist.

5. Vorrichtung nach einem der vorheraehenden Ansprüche, **dadurch gekennzeichnet, dass** die dritte Verarbeitungseinheit (122, 123) zur Bestimmung des Verhältnisses der Werte der spektralen Leistungsdichte in den vorbestimmten Frequenzbereichen eine Integratorstufe (122) zur Integration der Frequenzanteile innerhalb der beiden fest programmierten Bereiche von 0,05 bis 0,15 Hz ("LF") sowie von 0,15 bis 0,4 Hz ("HF") und eine mit dem Ausgang der Intearatorstufe (121) verbundene Arithmetikstufe (123) zur Bildung des Quotienten aus den beiden aktuellen Integralwerten aufweist.

6. Vorrichtung nach einem der vorhergehenden Ansprüche, **dadurch gekennzeichnet, dass** der Frequenzanalysator (120) zur Ausführung einer autoregressiven Spektralanalyse ausgebildet ist.

7. Vorrichtung nach Anspruch 6, **dadurch gekennzeichnet, dass** der Frequenzanalysator (120) zur Ausführung einer autoregressiven Spektralanalyse der Ordnung 20 ausgebildet ist.

8. Implantierbares Herztherapiegerät (100) mit einer Vorrichtung nach einem der vorhergehenden Ansprüche.

9. Implantierbares Herztherapiegerät nach Anspruch 8, **gekennzeichnet durch** die Ausbildung als ratenadaptiver Herzschrittmacher (100), mit einer Ratenfestlegungsstufe (115) zur Berechnung einer Stimulationsrate, die einen mindestens indirekt mit dem Ausgang der Vergleicher- und Bewertungseinheit (117) verbundenen Eingang zum Empfang des Lagesignals aufweist und zur Verarbeitung des Lagesignals bei der Berechnung der Stimulationsrate ausgebildet ist.

## Claims

1. Apparatus (100A) for detecting the body position of a human being including
a sensor (E, 101) for morphology-faithful detection of a physiological signal, which can be arranged in the heart (H) or a vessel near the heart,
**characterised by**
a control unit (104) for actuation of the sensor at predetermined moments in time, which is connected to a detector (E) for a physiological event or a stimulation signal generator (102) and is adapted to take off a signal detected by the sensor in relation to the physiological event or the stimulation event,
a first processing unit (105 to 108) connected at the input side to the sensor for determining a value of a signal parameter from the morphology of the detected signal in accordance with a predetermined signal processing rule,
a signal parameter memory (118) for storing signal parameter values determined in the past,
a second processing unit (119) connected at the input side to the signal parameter memory for ascertaining a fluctuation curve in respect of the stored signal parameter values,
a frequency analyser (120) connected at the input side to the second processing unit for obtaining a spectral power density from the fluctuation curve,
a third processing unit (122, 123) connected at the input side to the frequency analyser for determining the ratios of the values of the spectral power density in at least two predetermined frequency ranges, and
a comparator and evaluation unit (117) connected at the input side to the third processing unit for the output of a position signal in dependence on the ascertained ratio value.

2. Apparatus according to claim 1 **characterised in that** the sensor has an electrode (E) which can be arranged intracardially and a filter and amplifier stage (101) adapted for detecting the morphology of an intracardial ECG or impedance signal.

3. Apparatus according to claim 2 **characterised in that** the electrode is a unipolar electrode.

4. Apparatus according to one of the preceding claims **characterised in that** the physiological signal is the intracardial impedance or the ventricular-evoked stimulus response VER.

5. Apparatus according to one of the preceding claims **characterised in that** the third processing unit (122, 123) for determining the ratio of the values of the spectral power density in the predetermined frequency ranges has an integrator stage (122) for integration of the frequency components within the two fixedly programmed ranges of from 0.05 to 0.15 Hz ('LF') and from 0.15 to 0.4 Hz ('HF') and an arithmetic stage (123) connected to the output of the integrator stage (121) for forming the quotient of the two current integral values.

6. Apparatus according to one of the preceding claims **characterised in that** the frequency analyser (120) is adapted to effect autoregressive spectral analysis.

7. Apparatus according to claim 6 **characterised in that** the frequency analyser (120) is adapted to effect autoregressive spectral analysis of the order 20.

8. An implantable cardiac therapy device (100) having an apparatus according to one of the preceding claims.

9. An implantable cardiac therapy device according to claim 8 **characterised by** being in the form of a rate-adaptive cardiac pacemaker (100) with a rate establishing stage (115) for calculating a stimulation rate, which has an input connected at least indirectly to the output of the comparator and evaluation unit (117) for receiving the position signal and which is adapted to process the position signal upon calculation of the stimulation rate.

## Revendications

1. Dispositif (100A) en vue de la détection de la posture d'un être humain comprenant un capteur (E, 101) en vue de la réception relative à la morphologie d'un signal physiologique, qui peut être disposé dans le coeur (H) ou dans un vaisseau proche du coeur,
**caractérisé en ce que**
une unité de commande (104) en vue de l'excitation du capteur à des moments prédéterminés, qui est formée et reliée à un détecteur (E) pour un résultat physiologique ou à un générateur de signal de stimulation, afin de prélever un signal détecté par le capteur se rapportant au résultat physiologique ou au résultat de stimulation,
une première unité de traitement (105 à 108) reliée au capteur côté entrée en vue de la fixation d'une valeur d'un paramètre de signal à partir de la morphologie du signal détecté selon une disposition de traitement de signal prédéterminée,
une mémoire de paramètre de signal (118) en vue de la mémorisation de valeurs de paramètre de signal déterminées dans le passé,
une seconde unité de traitement (119) reliée à la mémoire de paramètre de signal côté entrée en vue de la détermination d'une courbe d'oscillation de la valeur de paramètre de signal mémorisée,
un analyseur de fréquence (120) relié à la seconde unité de traitement côté entrée en vue de la production d'une puissance volumique spectrale à partir de la courbe d'oscillation,
une troisième unité de traitement (122, 123) reliée à l'analyseur de fréquence en vue de la fixation de la proportion des valeurs de la puissance volumique spectrale dans au moins deux domaines de fréquence prédéterminés et
une unité de comparaison et d'évaluation (117) reliée à la troisième unité de traitement côté entrée en vue de la délivrance d'un signal de position selon la valeur de proportion déterminée.

2. Dispositif selon la revendication 1, **caractérisé en ce que** le capteur (E) qui peut être disposé de façon intracardiaque et présente un étage de filtre et d'amplificateur (101) en vue de la détection de la morphologie d'un signal d'ECG intracardiaque ou d'impédance.

3. Dispositif selon la revendication 2, **caractérisé en ce que** l'électrode est une électrode unipolaire.

4. Dispositif selon l'une des revendications précédentes, **caractérisé en ce que** le signal physiologique est l'impédance intracardiaque ou la réponse de stimulation évoquée ventriculaire VER.

5. Dispositif selon l'une des revendications précédentes, **caractérisé en ce que** la troisième unité de traitement (122, 123), en vue de la fixation de la proportion des valeurs de la puissance volumique spectrale dans les domaines de fréquence prédéterminés, présente un étage d'intégrateur (122) en vue de l'intégration de la part de fréquence à l'intérieur des deux domaines fermement programmés de 0,05 à 0,15 Hz ("LF") ainsi que de 0,15 à 0,4 Hz ("HF") et un étage d'arithmétique (123) relié à la sortie de l'étage d'intégrateur (121) en vue de la formation du quotient à partir des deux valeurs intégrales actuelles.

6. Dispositif selon l'une des revendications précédentes, **caractérisé en ce que** l'analyseur de fréquence (120) est réalisé en vue de la réalisation d'une analyse spectrale autorégressive.

7. Dispositif selon la revendication 6, **caractérisé en ce que** l'analyseur de fréquence (120) est réalisé en vue de la réalisation d'une analyse spectrale autorégressive d'ordre 20.

8. Appareil de thérapie cardiaque implantable (100) muni d'un dispositif selon l'une des revendications précédentes.

9. Appareil de thérapie cardiaque implantable selon la revendication 8, **caractérisé par** la réalisation en tant que stimulateur cardiaque s'adaptant à l'effort (100), avec un étage de définition d'effort (115) en vue du calcul d'un taux de stimulation, qui présente une entrée reliée au moins indirectement à la sortie de l'unité de comparaison et d'évaluation (117) en vue de la réception du signal de position et est réalisé en vue du traitement du signal de position par le calcul du taux de stimulation.
